# FASCICULE DE BREVET EUROPEEN

(11) **EP 2 574 368 B1**
(45) Date de publication et mention de la délivrance du brevet: **24.12.2014**
(21) Numéro de dépôt: 12172599.8
(22) Date de dépôt: 19.06.2012
(51) Int. Cl.: A61N 1/05

(54) **Sonde de stimulation épicardique multizone**
Sonde zur Multizonen-Epikardstimulation
Multizone epicardial stimulation probe

(30) Priorité: 30.09.2011 FR 1158882
(43) Date de publication de la demande: 03.04.2013
(73) Titulaire: Sorin CRM SAS, 92140 Clamart Cedex (FR)
(72) Inventeur: Ollivier, Jean-François, 91190 VILLIERS LE BACLE (FR)
(74) Mandataire: Dupuis-Latour, Dominique

(56) Documents cités:
- WO-A1-2005/039690
- US-A1- 2007 043 412
- US-A1- 2009 157 136

## Description

L'invention concerne de façon générale les "dispositifs médicaux implantables actifs" tels que définis par la directive 90/385/CEE du 20 juin 1990 du Conseil des communautés européennes.

Cette définition inclut en particulier les implants cardiaques chargés de surveiller l'activité cardiaque et de générer des impulsions de stimulation, de resynchronisation, de défibrillation et/ou de cardioversion en cas de trouble du rythme détecté par l'appareil.

Elle concerne plus précisément les sondes dites "épicardiques" qui sont directement fixées sur la paroi extérieure du myocarde, par opposition aux sondes dites "endocardiaques" qui sont logées dans une cavité du coeur où elles y sont introduites via le réseau veineux, et aux sonde "coronaires" implantées dans le réseau coronarien du coeur.

Les sondes épicardiques peuvent être notamment prescrites pour la stimulation du ventricule gauche, en alternative aux sondes de stimulation implantées via le sinus coronaire, qui nécessitent pour leur pose une approche délicate et ne sont pas dénuées d'inconvénients divers.

Toutefois, à la différence des sondes endocavitaires introduites via le réseau veineux (voir par exemple le US 2009/0157136 A1), la mise en place d'une sonde épicardique est une opération très lourde, nécessitant généralement une anesthésie générale et le recours à des techniques chirurgicales très invasives. Il est en effet nécessaire qu'un chirurgien incise le thorax de manière à ménager un accès au "sac péricardique" (le péricarde étant l'enveloppe fibro-séreuse qui enveloppe le coeur), puis au myocarde lui-même, afin de pouvoir fixer la sonde sur la paroi externe de ce dernier, par suturation ou par vissage.

Pour cette raison, la pose d'une sonde épicardique, bien que constituant une technique connue, est souvent considérée comme une solution de repli en cas d'échec d'implantation d'une sonde via le sinus coronaire.

Au surplus, une sonde épicardique ne fournit souvent que des résultats médiocres, en particulier au niveau des performances électriques. Plusieurs types de sondes épicardiques ont été proposés, mais toutes présentent des inconvénients assez sérieux.

Un premier type de sonde utilise une électrode plaquée contre la paroi de l'épicarde, où elle est maintenue par suturation. Ces sondes sont très stables, mais elles nécessitent un large accès pour permettre la suture par le chirurgien, et la zone de pose possible est très limitée, car restreinte à la proximité de l'incision thoracique.

Une variante de ce type de sonde consiste à prévoir un corps de sonde se divisant en deux branches distinctes, avec à l'extrémité distale de chacune un support d'électrode de stimulation, à suturer sur la paroi cardiaque. Ce support est prolongé par un fil résorbable terminé par une aiguille : lors de l'intervention, après avoir suturé l'électrode et son support, le chirurgien enfouit le fil résorbable au moyen de l'aiguille (qui sera par la suite retirée) en exerçant une traction sur le fil. Cet effort de traction permanente assure un excellent contact de l'électrode, sollicitée de façon positive contre la paroi cardiaque. La présence de deux électrodes distinctes permet en outre d'appliquer une stimulation bipolaire, sur une zone quelque peu élargie.

On a également proposé un autre type de sonde, munie d'une vis hélicoïdale d'ancrage dans la paroi du myocarde. Le vissage peut se faire directement, mais la zone de travail est alors limitée de la même façon que pour une sonde suturée. Il peut également se faire au moyen d'un instrument de pose spécifique comportant une tête articulée sur laquelle est montée la sonde, mais la zone de pose possible, bien qu'élargie, sera toutefois limitée par la rigidité du tube support de l'instrument et par le diamètre important de celui-ci, que le chirurgien doit faire progresser dans l'espace péricardique courbe.

De plus, sur le plan mécanique, les vis des sondes actuelles sont très largement dimensionnées, en raison des contraintes mécaniques importantes exercées sur la vis lors de la pose, du fait des fortes amplitudes des déplacements et des tractions radiales exercées. Ces vis largement dimensionnées sont relativement traumatiques pour les tissus, avec risque de création locale de fibroses réactionnelles.

L'inconvénient commun à toutes ces sondes, outre le caractère fortement invasif de l'intervention, réside dans leurs faibles performances électriques, notamment en raison de la taille importante de l'électrode suturée (qui doit assurer un contact satisfaisant avec la paroi cardiaque) ou des dimensions importantes de la vis (nécessaire pour supporter les conditions de pose). Or une électrode de taille importante ne procure pas une densité de courant satisfaisante, ce qui va au détriment de l'efficacité de la stimulation.

En outre, la stimulation reste ponctuelle (un seul site de stimulation), avec un double inconvénient :
- une moindre efficacité de la stimulation par rapport à une configuration multisite ; et
- le risque que le site choisi ne soit pas le plus efficace, ou que compte tenu du remodelage cardiaque, le site initialement choisi devienne au fil du temps moins efficace ; bien entendu, compte tenu de la lourdeur de l'opération, il ne peut pas être envisagé de réintervenir sur le patient pour tenter d'améliorer la situation en recherchant un éventuel autre site qui serait plus efficace que le site initialement choisi.

Un dernier inconvénient des sondes épicardiques actuelles, en particulier des sondes à vis, est leur quasi-impossibilité d'extraction une fois qu'elles ont été posées.

Pour toutes ces raisons, l'usage des sondes épicardiques reste encore peu développé, et ces sondes ne sont généralement utilisées qu'en dernier recours lorsque les autres techniques ne sont pas praticables. L'invention vise à s'affranchir des diverses limitations exposées ci-dessus, en proposant une sonde de stimulation épicardique dont la partie active :
- garantisse un contact électrique d'excellente qualité et durable avec les tissus à stimuler ;
- et surtout, multiplie ou élargisse la zone de stimulation, permettant ainsi (contrairement aux sondes traditionnelles) de stimuler simultanément plusieurs régions de l'épicarde en améliorant de ce fait l'efficacité de la stimulation.

À ce dernier égard, on a en effet constaté avec les dispositifs de resynchronisation cardiaque que la multiplication des points de stimulation sur le ventricule gauche est un facteur permettant d'améliorer de façon substantielle la qualité de la resynchronisation.

Le US 2007/0043412 A1 propose un dispositif mettant en oeuvre plusieurs électrodes (d'un type en lui-même conventionnel) implantées en différents endroits de l'épicarde de manière à constituer un réseau de ces électrodes. Du point de vue électrique, chaque électrode est pourvue d'un conducteur de liaison propre, et les conducteurs respectifs sont connectés ensemble à un conducteur commun relié à son autre extrémité au générateur. Les impulsions de stimulation peuvent être ainsi diffusées simultanément sur plusieurs électrodes en plusieurs points du myocarde, correspondant aux points d'implantation des électrodes.

Le WO 2005/039690 A1, quant à lui, décrit une autre configuration, où deux électrodes épicardiques à vis sont implantées face à chacun des deux ventricules, chaque électrode étant reliée au générateur par un conducteur propre.

Avec ces dispositifs, dans la mesure où les électrodes utilisées sont de type conventionnel (typiquement, des électrodes à vis), les problèmes évoqués ci-dessus liés à la nature des électrodes subsistent, à savoir : difficulté de pose avec recours à des instruments complexes notamment pour le vissage *in situ* une fois la position d'implantation atteinte ; nécessité d'une intervention fortement invasive ; caractère traumatique pour les tissus avec risque de création locale de fibroses réactionnelles des vis largement dimensionnées en raison des contraintes mécaniques ; faibles performances électriques du fait de la taille importante de l'électrode, qui ne procure pas une densité de courant satisfaisante, au détriment de l'efficacité de la stimulation.

Le but de l'invention est de proposer une sonde épicardique multipliant les points de stimulation, qui soit de structure simple (donc peu coûteuse à fabriquer et présentant une fiabilité maximale), et qui, surtout, puisse en outre être implantée par des techniques opératoires habituellement pratiquées par les chirurgiens.

Essentiellement, la solution de l'invention consiste à distribuer à la surface du muscle cardiaque, ou dans celui-ci, un réseau de micro-électrodes elles-mêmes portées par une pluralité de microcâbles.

Ces microcâbles, très souples, rayonnent à partir d'un boîtier répartiteur ou *hub* commun monté à l'extrémité d'un corps de sonde épicardique assurant la distribution du courant vers les divers microcâbles, avec possibilité de stimulation bipolaire et/ou de multiplexage des différents microcâbles. Le boîtier répartiteur dispose de ses propres moyens de fixation à la paroi cardiaque (suture ou vis), et il est lui-même dépourvu d'électrode. Plus précisément, l'invention porte sur une sonde épicardique de stimulation comprenant, de manière en elle-même connue notamment d'après le US 2007/0043412 A1 précité : un corps de sonde avec une gaine en matériau déformable, enfermant au moins un conducteur de liaison ; côté proximal, un connecteur de couplage à un générateur de dispositif médical implantable actif ; côté distal, des moyens d'ancrage à une paroi de l'épicarde et une partie active comprenant une pluralité d'électrodes de stimulation, apte à venir en contact avec, ou à pénétrer dans, la paroi de l'épicarde ; un boîtier répartiteur, placé à l'extrémité du corps de sonde ; et un réseau de conducteurs flexibles électriquement isolés rayonnant à partir du boîtier répartiteur et s'étendant entre une extrémité proximale reliée au boîtier répartiteur et une extrémité distale libre éloignée du boîtier répartiteur, et électriquement reliés au niveau du boîtier répartiteur à un conducteur de liaison du corps de sonde.

De façon caractéristique de l'invention, les conducteurs flexibles sont des éléments de type microcâble de diamètre au plus égal à 2 French (0,66 mm). Chaque microcâble comprend au moins une zone ponctuellement dénudée, et chacune de ces zones forme l'une des électrodes de stimulation, les électrodes de stimulation d'un même microcâble étant électriquement reliées ensemble. Chaque microcâble comporte en outre au moins un élément allongé transversal s'étendant en formant un angle par rapport à la direction principale du microcâble, et apte à pénétrer dans la paroi de l'épicarde.

Selon diverses caractéristiques subsidiaires avantageuses :
- ledit au moins un élément allongé transversal est une boucle d'enfouissement formée par une courbure du microcâble, ou une extension libre formée en dérivation par rapport au microcâble ;
- une extrémité radialement distale dudit au moins un élément allongé transversal porte l'une desdites zones ponctuellement dénudées, de manière à permettre une stimulation en profondeur de l'épicarde ;
- chaque microcâble comporte une succession de parties allongées s'étendant suivant la direction principale du microcâble, séparées par des boucles de compliance s'étendant dans une direction transversale par rapport au microcâble et aptes à procurer au microcâble une flexibilité et une extensibilité dans le sens longitudinal ;
- les zones ponctuellement dénudées de chaque microcâble sont formées à une extrémité radialement distale des boucles de compliance, de manière à permettre une stimulation en surface de l'épicarde ;
- les boucles de compliance et les éléments allongés transversaux s'étendent dans des plans respectifs différents, faisant un angle entre eux ;
- la longueur de chaque microcâble, à l'état déployé, est comprise entre 5 et 80 mm ;
- le boîtier répartiteur porte les moyens d'ancrage à une paroi de l'épicarde ;
- la surface totale exposée des zones ponctuellement dénudées de chaque microcâble est d'au plus 10 mm² ;
- la longueur en direction longitudinale de chaque zone ponctuellement dénudée de chaque microcâble est d'au plus 10 mm ;
- le corps de sonde enferme une pluralité de conducteurs de liaison distincts, et la sonde comporte une pluralité correspondante de microcâbles électriquement isolés entre eux et respectivement reliés aux conducteurs de liaison, de manière à permettre une stimulation bipolaire ou multipolaire entre des parties ponctuellement dénudées de microcâbles respectifs différents ;
- la sonde comporte une pluralité correspondante de microcâbles électriquement isolés entre eux, et le boîtier répartiteur comporte des moyens de commutation contrôlée entre, d'une part, un microcâble sélectionné parmi plusieurs de la pluralité de microcâbles et, d'autre part, un conducteur de liaison commun du corps de sonde, de manière à permettre une sélection parmi les parties ponctuellement dénudées de microcâbles respectifs différents ;
- le microcâble est formé d'une pluralité de brins toronnés ensemble, dont au moins certains sont des brins incorporant une âme en matériau radio-opaque de type platine-iridium ou tantale enveloppée dans une gaine en matériau mécaniquement endurant de type NiTi ou inox, ou inversement ;
- le microcâble comprend une structure multifilaire revêtue par un matériau isolant, par exemple du parylène ou une gaine de type PET ou PMMA, dans lequel les régions dénudées sont formées en ménageant des ouvertures par ablation le long du microcâble ;
- l'extrémité du microcâble est dotée d'une aiguille courbe pour le piquage et l'enfouissement du microcâble lors de la pose de la sonde, cette aiguille étant apte à être ultérieurement dissociée du microcâble par découpe (un fil résorbable peut être prévu à cet effet entre l'aiguille et l'extrémité du microcâble).

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 illustre de façon schématique une sonde épicardique selon l'invention, dans un premier mode de réalisation.
La Figure 2 est homologue de la Figure 1, pour un second mode de réalisation où chaque microcâble est doté d'une pluralité d'électrodes de stimulation.
La Figure 3 est homologue de la Figure 1, pour un troisième mode de réalisation mettant en oeuvre un système de multiplexage entre les différents microcâbles de la sonde.
La Figure 4 montre, en perspective, une première configuration possible d'un microcâble pour une sonde selon l'invention.
La Figure 5 illustre le microcâble de la Figure 4, vu en projection dans les deux plans orthogonaux P₁ et P₂.
La Figure 6 illustre une variante de la Figure 5, avec un positionnement différent des électrodes de stimulation.
La Figure 7 est une vue schématique en plan d'une sonde épicardique selon l'invention, implantée, avec une configuration de microcâble telle que celle illustrée Figure 5.
La Figure 8 est homologue de la Figure 7, vue en coupe transversale au travers de la paroi cardiaque.
La Figure 9 montre, en perspective, une deuxième configuration possible d'un microcâble pour une sonde selon l'invention.
La Figure 10 illustre le microcâble de la Figure 9, vu en projection dans les deux plans orthogonaux P₁ et P₂.
La Figure 11 est une vue schématique, en coupe transversale au travers de la paroi cardiaque, d'une sonde épicardique selon l'invention, implantée, avec une configuration de microcâble telle que celle illustrée Figure 9.

On va maintenant décrire plusieurs exemples de réalisation de la sonde épicardique de l'invention.

La sonde épicardique 10 selon l'invention comprend, essentiellement, un corps de sonde 12 terminé à son extrémité proximale par un connecteur 14 de type conventionnel (de type IS-1 ou IS-4).

A son extrémité distale, le corps de sonde est doté d'un boîtier répartiteur 16 ou *hub* pourvu de moyens de fixation d'un type connu (suture ou vis), mais non stimulants : en effet, l'invention ne prévoit pas que ce boîtier porte une électrode de stimulation. Le boîtier 16 porte éventuellement une collerette en film perforé permettant de renforcer l'ancrage par développement d'une fibrose. Le boîtier 16 peut présenter par exemple une forme cylindrique aplatie, avec un diamètre typique de 6 mm pour une hauteur de 4 mm.

De façon caractéristique de l'invention, une pluralité de microcâbles 18 sont connectés au boîtier répartiteur 16 par l'une de leurs extrémités, l'autre extrémité étant libre (éventuellement munie d'une aiguille destinée au chirurgien, qui sera retirée une fois l'implantation réalisée). Le boîtier répartiteur 16 porte typiquement de six à huit microcâbles 18, mais ce nombre n'est donné qu'à titre d'exemple et n'est en aucune façon limitatif. La longueur de chaque microcâble, à l'état déployé, est typiquement comprise entre 5 et 80 mm.

Le boîtier répartiteur 16 assure la liaison physique et électrique de chaque microcâble avec un conducteur correspondant interne du corps de sonde 12,ce conducteur s'étendant jusqu'au connecteur 14.

Par "microcâble" on entendra un câble de très faible diamètre, au plus égal à 2 French (0,66 mm), typiquement de l'ordre de 1 French (0,33 mm). Chaque microcâble est formé d'un conducteur métallique électriquement isolé, à l'exception d'au moins une zone 20 ponctuellement dénudée ou "fenêtre", formant une électrode de stimulation correspondante (schématisée par une étoile sur la figure).

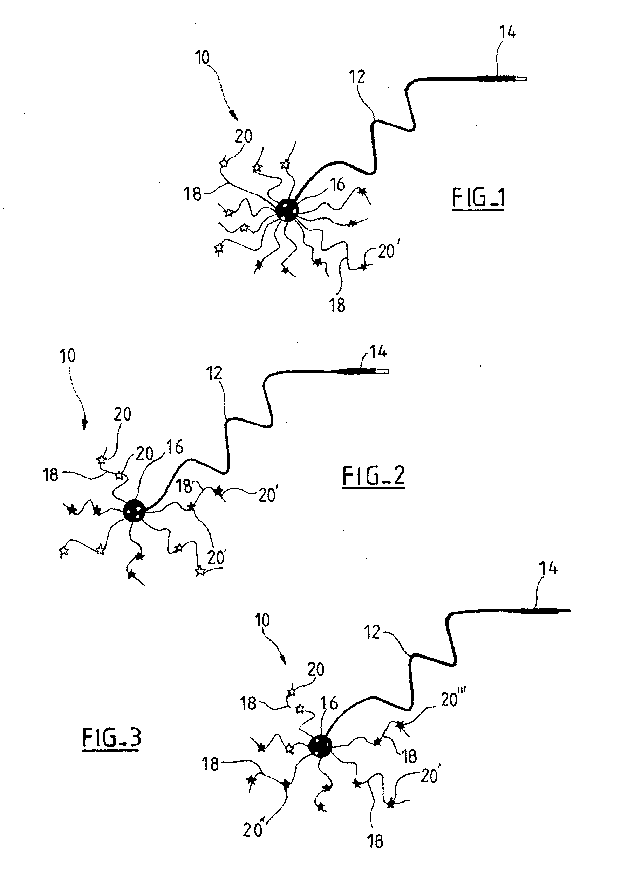

Dans le mode de réalisation illustré Figure 1, chaque microcâble porte une seule de ces zones ponctuellement dénudées 20, 20' formant électrode (dans la suite, on utilisera indifféremment les termes de "zone ponctuellement dénudée", "électrode" ou "fenêtre").

Une partie des microcâbles porte des électrodes 20 reliées ensemble dans le boîtier 16 à un premier conducteur du corps de sonde, l'autre partie des microcâbles portant des électrodes 20' reliées ensemble et à un autre conducteur, différent du premier, du corps de sonde. Il est ainsi possible d'appliquer une stimulation bipolaire entre, d'une part, les électrodes 20 (schématisées par des étoiles blanches) et les électrodes 20' (schématisées par des étoiles noires).

Dans le mode de réalisation de la Figure 2, chaque microcâble 18 porte une pluralité d'électrodes, par exemple deux électrodes 20 ou deux électrodes 20', ce qui permet d'élargir la zone de stimulation le long d'un même microcâble.

La Figure 3 illustre un troisième mode de réalisation, dans lequel les microcâbles 18 portent des électrodes 20, 20', 20", 20"'... et dans lequel chaque microcâble est relié à l'entrée d'un module de multiplexage incorporé au boîtier répartiteur 16. Une commande appropriée envoyée au boîtier de multiplexage permet de sélectionner à volonté l'une ou l'autre série d'électrodes 20, 20', 20", 20"'... afin de tester les sites de stimulation possibles et choisir celui ou ceux procurant les meilleurs résultats du point de vue physiologique.

Le système de multiplexage peut être par exemple celui décrit notamment dans les EP 1 938 861 A1 et EP 2 082 684 A1 (ELA Medical), qui permet en particulier de mettre en oeuvre le concept de "repositionnement électronique" visant à diriger ou rediriger le champ électrique entre différentes électrodes en sélectionnant, parmi les diverses configurations possibles, celles procurant la meilleure efficacité sur le plan électrique et hémodynamique. Cette technologie permet en outre de gérer les évolutions du comportement hémodynamique (remodelage inverse), simplement par re-programmation du générateur par voie télémétrique à travers la peau, donc sans réintervention chirurgicale lourde.

Pour multiplier les zones de stimulation, une variante consiste à prévoir par exemple quatre microcâbles ou groupes de microcâbles indépendants reliés chacun à quatre conducteurs distincts à l'intérieur du corps de sonde 12. Le connecteur 14 est alors un connecteur approprié, par exemple de type IS-4, permettant ainsi de bénéficier de quatre zones de stimulation indépendantes.

Dans tous les cas, les parties dénudées 20 de chaque microcâble 18 forment une succession d'électrodes individuelles, constituant ensemble un ensemble d'électrodes reliées en série. Ceci permet de multiplier les points de contact avec la paroi cardiaque et d'assurer ainsi une diffusion multizone de l'énergie de stimulation en plusieurs points de l'épicarde et donc du ventricule gauche.

De préférence, la surface individuelle de chaque électrode est d'au plus 1 mm², ce qui permet d'en disposer plusieurs sans dépasser une surface totale cumulée de 10 mm². Du fait de la faible surface active cumulée, on bénéficie ainsi des avantages d'une sonde dite à "haute densité de courant", avec à la fois une meilleure efficacité physiologique de la stimulation et une moindre consommation d'énergie - ceci tout en maximisant les chances de contact physique, donc électrique, des électrodes avec les tissus excitables, du fait de la multiplication de ces électrodes.

En ce qui concerne le microcâble 18 utilisé dans ces divers modes de réalisation, l'âme de celui-ci est avantageusement réalisée à base de nitinol (alliage NiTi) ou de MP35N-LT (35% Ni, 35% Co, 20% Cr et 10% Mo), matériaux dont l'avantage essentiel est leur extrême endurance en fatigue, avec enrobage par une gaine en platine iridié. Il s'agit d'obtenir nativement la résistance à la corrosion au niveau des électrodes, tout en garantissant la résistance à la fatigue impérativement requise.

Plus précisément, la structure du microcâble est avantageusement une structure multifilaire dans laquelle chaque brin est de préférence constitué d'une âme en platine-iridium gainée par une épaisseur de nitinol ou de MP35N-LT - ou inversement, de manière à optimiser la réponse aux exigences à la fois de résistance à la corrosion et à la fatigue.

L'ensemble peut alors être revêtu :
- soit par une fine couche de parylène (par exemple de type C). Dans ce cas, des fenêtres plus ou moins complexes sont aménagées le long du microcâble, par exemple par ablation plasma, pour former les électrodes 20 ; pour améliorer les performances électriques, ces zones peuvent être en outre revêtues par exemple de nitrure de titane ;
- soit par un tube polyuréthanne interrompu aux endroits des électrodes 20 ;
- soit par une ou plusieurs couches constituées de tubes en PET (polyéthylène téréphtalate), polymère fluoré, PMMA (polyméthacrylate de méthyle), PEEK (polyétheréthercétone), polyimide ou autre matériau similaire approprié.

Une telle structure de microcâble, sans lumière interne et avec plusieurs microfils tressés ensemble, est capable d'assurer à la fois l'endurance (à l'encontre des mouvements cardiaques) et la résistance aux sollicitations notamment liées à la pose.

Un autre avantage de cette solution, particulièrement significatif, résulte du caractère très souple et flottant (*floppy*) du microcâble, qui lui confère une excellente atraumaticité. En chronique, un tel microcâble agresse peu les tissus et préserve donc les cellules à proximité immédiate des électrodes : on peut donc espérer une bonne performance électrique y compris sur le long terme, contrairement aux sondes épicardiques traditionnelles, qui sont bien plus traumatiques.

Ces types de microcâbles sont disponibles par exemple auprès de la société Fort Wayne Metals Inc., Fort Wayne, USA, et sont utilisés dans le domaine médical notamment pour réaliser des conducteurs de défibrillation - toutefois selon un arrangement de matériaux différent : dans ces applications connues, la structure est une structure multifilaire dans laquelle chaque brin inclut une âme en argent (pour améliorer la conductivité) gainée par une épaisseur d'inox ; ces microstructures, isolées ou non, sont ensuite incorporées dans un corps de sonde multi-lumière de construction classique.

Comme alternative, il est néanmoins possible de disposer un brin en platine-iridium au centre d'une structure multifilaire de type 1 x7, le brin le plus fragile étant alors enlacé par les brins externes plus endurants.

Enfin, le platine-iridium peut être remplacé par tout matériau radio-opaque tel que le tantale.

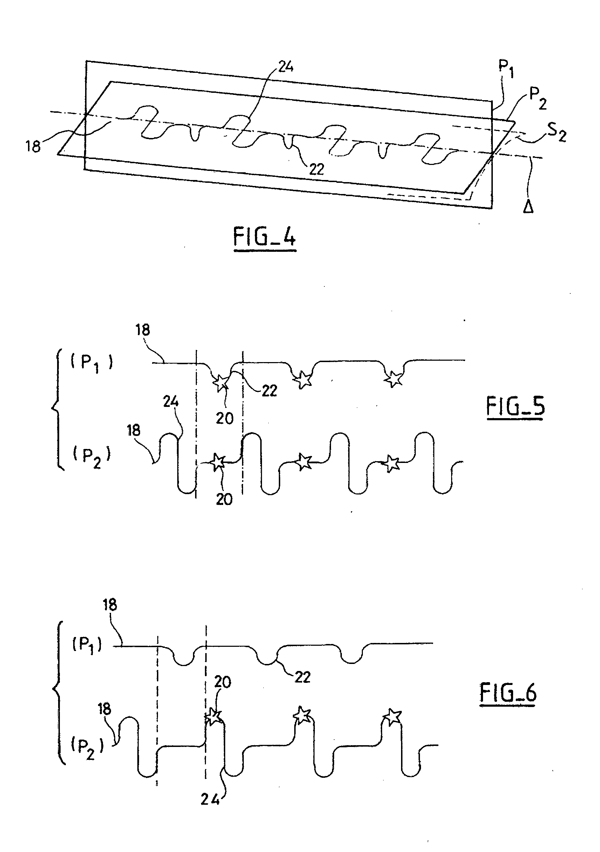

On va maintenant exposer diverses conformations géométriques possibles des microcâbles 18 (préformés à la fabrication), en référence aux Figures 4 à 11.

Sur les Figures 4 et 5 on a illustré une première configuration possible, dans laquelle le microcâble 18 comporte une série de portions ondulées alternées 22, 24, avec un ou plusieurs éléments allongés transversaux 22 s'étendant dans un premier plan P₁ et des boucles de compliance 24 s'étendant dans un second plan P₂ orthogonal au plan P₁, l'intersection de ces deux plans P₁ et P₂ se confondant avec la direction principale Δ (sens longitudinal) du microcâble.

Les éléments allongés transversaux 22 constituent des boucles d'enfouissement permettant de faire pénétrer dans l'épaisseur de l'épicarde les parties localement dénudées 20 formant les électrodes de stimulation, qui sont localisées au sommet des boucles d'enfouissement 22.

Les boucles de compliance 24, par exemple réalisées sous la forme de deux demi-périodes successives de sinusoïde ou d'une forme approchante, permettent d'éviter la transmission des contraintes cycliques, résultant du battement du coeur, aux zones stimulantes formées par les électrodes 20 au sommet des boucles d'enfouissement 22.

On notera que la simplicité de la structure - microcâble isolé avec des zones ponctuellement dénudées formant les électrodes - permet de prévoir sans problème une électrode au sommet d'une ondulation (préformée) du microcâble, ce qui serait beaucoup plus délicat avec des structures conventionnelles, pour lesquelles on considère que les zones de courbure maximale sont *a priori* les plus sollicitées, ce qui conduit à éviter d'y localiser les électrodes.

De plus, la localisation au sommet des boucles d'enfouissement 22 des parties dénudées 20 offre la possibilité de sectoriser les électrodes, c'est-à-dire de faire en sorte que, vues en section droite, elles ne s'étendent pas sur toute la périphérie du microcâble, mais seulement sur un secteur angulaire situé du côté de la face extérieure de la courbure, c'est-à-dire du côté tourné vers les tissus avec lesquels elles viendront en contact. Il est de ce fait possible de garder isolé une grande partie de secteur angulaire, ce qui limite encore les surfaces stimulantes, avec pour conséquence directe les avantages exposés plus haut en termes d'accroissement de la densité de courant.

L'utilisation d'une électrode enfouie correspondant à la configuration des Figures 4 et 5 diminue le risque de stimulation phrénique, et la stimulation profonde qu'elle permet assure une meilleure efficacité du point de vue électrique et hémodynamique.

En variante, il est toutefois possible de prévoir une autre configuration, telle que celle illustrée Figure 6, où les électrodes 20 sont disposées au sommet des boucles de compliance 24. Les boucles d'enfouissement 22 sont alors utilisées uniquement comme points d'ancrage du microcâble à la paroi cardiaque.

Avec cette deuxième configuration, la surface dans laquelle s'étendent les boucles de compliance 24 est de préférence une surface courbée S₂ (Figure 4) au lieu d'un plan P₂, avec une courbure dirigée vers la paroi (c'est-à-dire dans le sens des boucles d'enfouissement 22). Ceci permet de forcer le contact mécanique des électrodes 20 avec la surface du muscle, du fait de l'effet ressort vertical de la boucle de compliance 24 dès lors que la courbure de la surface S₂ est, à l'état libre, supérieure à celle du muscle cardiaque.

Avec de cette manière un contact de surface par l'électrode au lieu d'une électrode enfouie, on réduit les traumatismes subis par les tissus, ce qui peut augmenter la performance électrique. Pour réduire le risque de stimulation phrénique, il est possible de sectoriser la surface des électrodes de stimulation 20 situées au sommet des boucles de compliance 24, c'est-à-dire du côté tourné vers les tissus avec lesquels elles viendront en contact. Il est de ce fait possible de garder isolée une grande partie de secteur angulaire, ce qui limite encore les surfaces stimulantes, avec pour conséquence directe les avantages exposés plus haut en termes d'accroissement de la densité de courant.

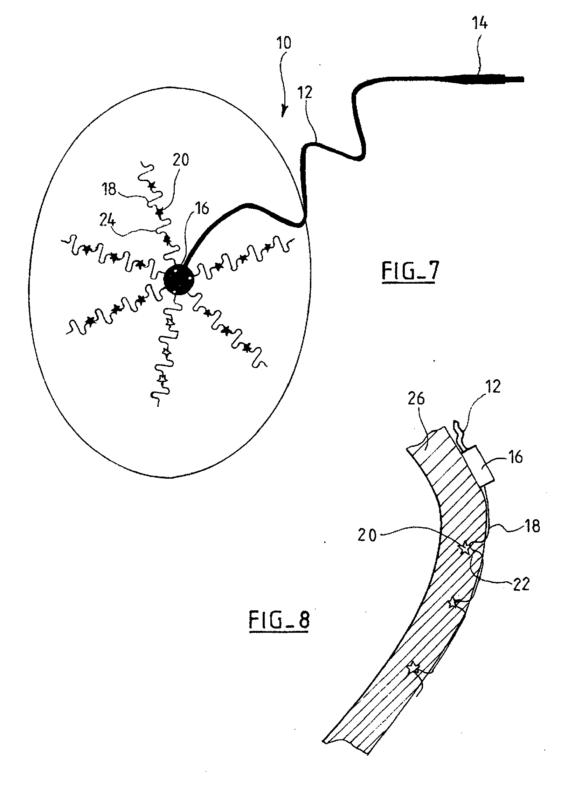

La Figure 7 et 8 sont des vues schématiques, respectivement en plan et en coupe transversale au travers de la paroi cardiaque 26, d'une sonde épicardique selon l'invention avec une configuration de microcâble telle que celle illustrée Figure 5, en situation implantée.

La pose de la sonde selon l'invention débute par la fixation préalable du boîtier répartiteur 16 sur la paroi cardiaque.

L'étape suivante consiste à disposer successivement les divers microcâbles sur la paroi cardiaque, avec enfouissement de la boucle ou des boucles 22 permettant d'assurer le maintien en position du microcâble. Ces boucles 22 portant (ou non) les électrodes de stimulation peuvent être enfouies dans le muscle par une série de piqures régulièrement espacées.

L'extrémité du microcâble sera dotée à cet effet, comme expliqué plus haut, d'une aiguille courbe, montée en usine, permettant de piquer/enfouir le microcâble à intervalles réguliers, l'aiguille étant coupée après enfouissement. L'extrémité coupée de ce dernier sera alors isolée par un dépôt de colle biocompatible. Pour éviter de créer une électrode supplémentaire à l'endroit de la coupe, il est possible d'intercaler un fil résorbable entre l'aiguille et l'extrémité du microcâble, la coupe s'effectuant alors sur le fil résorbable.

On soulignera que la configuration particulière de la sonde de l'invention est particulièrement bien adaptée à une intervention par des techniques de microchirurgie robotisée, en tirant notamment profit des capacités remarquables de cette technologie d'opérer des "microcoutures" de façon automatique sous contrôle à distance par le chirurgien.

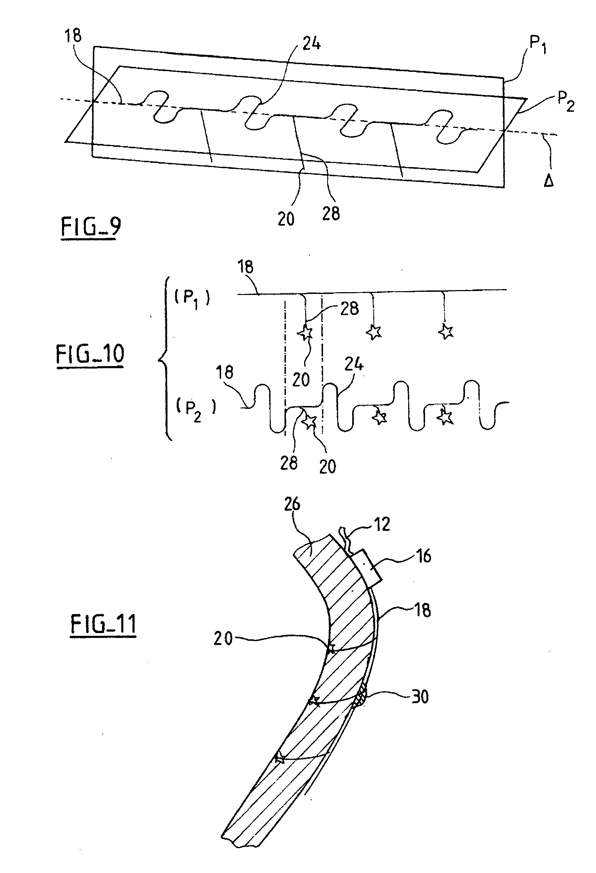

Les Figures 9, 10 et 11 sont homologues des Figures 4, 5 et 8, respectivement, pour une variante de réalisation du microcâble dans laquelle les un ou plusieurs éléments allongés transversaux, au lieu d'être des boucles d'enfouissement, sont des extensions libres 28 sensiblement rectilignes portant à leur extrémité libre l'électrode de stimulation 20. Ces extensions 28 sont connectées à leur autre extrémité au microcâble 18 qui court sur la surface du muscle cardiaque (ce microcâble 18 étant bien entendu pourvu des boucles de compliance 24 permettant d'éviter la transmission de contraintes entre les extensions libres 28).

Pour la pose, chaque extension libre 28 peut être préalablement logée à l'intérieur d'une micro-aiguille sécable de ponction, permettant de réaliser l'enfouissement de l'extension libre stimulante 28 par enfoncement de l'aiguille puis retrait de celle-ci (grâce à sa sécabilité) une fois l'enfouissement réalisé.

Cette configuration de microcâble permet de délivrer une stimulation profonde du myocarde, proche d'une stimulation endocardiaque.

En fin d'intervention, l'émergence épicardique de l'extrémité libre est fixée à la paroi par une méthode de fixation connue telle que points de suture ou dépôt d'un point de colle 30 d'un adhésif chirurgical biocompatible tel que *Bioglue* (marque déposée).

## Revendications

1. Une sonde épicardique (10) de stimulation, comprenant :
- un corps de sonde (12) avec une gaine en matériau déformable, enfermant au moins un conducteur de liaison ;
- côté proximal, un connecteur (14) de couplage à un générateur de dispositif médical implantable actif ;
- côté distal, des moyens d'ancrage à une paroi de l'épicarde et une partie active comprenant une pluralité d'électrodes de stimulation, apte à venir en contact avec, ou à pénétrer dans, la paroi de l'épicarde ;
- un boîtier répartiteur (16), placé à l'extrémité du corps de sonde ; et
- un réseau de conducteurs flexibles électriquement isolés (18) rayonnant à partir du boîtier répartiteur et s'étendant entre une extrémité proximale reliée au boîtier répartiteur et une extrémité distale libre éloignée du boîtier répartiteur, et électriquement reliés au niveau du boîtier répartiteur à un conducteur de liaison du corps de sonde,
sonde **caractérisée en ce que** :
- lesdits conducteurs flexibles (18) sont des éléments de type microcâble de diamètre au plus égal à 2 French (0,66 mm) ;
- chaque microcâble comprend au moins une zone ponctuellement dénudée (20), chacune de ces zones formant l'une desdites électrodes de stimulation, les électrodes de stimulation d'un même microcâble étant électriquement reliées ensemble, et
- chaque microcâble comporte au moins un élément allongé transversal (22 ; 28) s'étendant en formant un angle par rapport à la direction principale du microcâble, et apte à pénétrer dans la paroi de l'épicarde.

2. La sonde épicardique de la revendication 1, dans laquelle ledit au moins un élément allongé transversal est une boucle d'enfouissement (22) formée par une courbure du microcâble.

3. La sonde épicardique de la revendication 1, dans laquelle ledit au moins un élément allongé transversal est une extension libre (28) formée en dérivation par rapport au microcâble.

4. La sonde épicardique de la revendication 1, dans laquelle une extrémité radialement distale dudit au moins un élément allongé transversal (22 ; 28) porte l'une desdites zones ponctuellement dénudées, de manière à permettre une stimulation en profondeur de l'épicarde.

5. La sonde épicardique de la revendication 1, dans laquelle chaque microcâble comporte une succession de parties allongées s'étendant suivant la direction principale du microcâble, séparées par des boucles de compliance (24) s'étendant dans une direction transversale par rapport au microcâble et aptes à procurer au microcâble une flexibilité et une extensibilité dans le sens longitudinal.

6. La sonde épicardique de la revendication 5, dans laquelle les zones ponctuellement dénudées (20) de chaque microcâble sont formées à une extrémité radialement distale des boucles de compliance (24), de manière à permettre une stimulation en surface de l'épicarde.

7. La sonde épicardique de la revendication 5, dans laquelle les boucles de compliance (24) et les éléments allongés transversaux (22 ; 28) s'étendent dans des plans respectifs différents (P₁, P₂), faisant un angle entre eux.

8. La sonde épicardique de la revendication 1, dans laquelle la longueur de chaque microcâble, à l'état déployé, est comprise entre 5 et 80 mm.

9. La sonde épicardique de la revendication 1, dans laquelle le boîtier répartiteur (16) porte les moyens d'ancrage à une paroi de l'épicarde.

10. La sonde épicardique de la revendication 1, dans laquelle la surface totale exposée des zones ponctuellement dénudées de chaque microcâble est d'au plus 10 mm².

11. La sonde épicardique de la revendication 1, dans laquelle la longueur en direction longitudinale de chaque zone ponctuellement dénudée de chaque microcâble est d'au plus 10 mm.

12. La sonde épicardique de la revendication 1, dans laquelle le corps de sonde enferme une pluralité de conducteurs de liaison distincts, et la sonde comporte une pluralité correspondante de microcâbles électriquement isolés entre eux et respectivement reliés aux conducteurs de liaison, de manière à permettre un stimulation bipolaire ou multipolaire entre des parties ponctuellement dénudées (20, 20') de microcâbles respectifs différents.

13. La sonde épicardique de la revendication 1, dans laquelle la sonde comporte une pluralité correspondante de microcâbles électriquement isolés entre eux, et le boîtier répartiteur comporte des moyens de commutation contrôlée entre, d'une part, un microcâble sélectionné parmi plusieurs de la pluralité de microcâbles et, d'autre part, un conducteur de liaison commun du corps de sonde, de manière à permettre un sélection parmi les parties ponctuellement dénudées (20, 20', 20", 20"') de microcâbles respectifs différents.

14. La sonde épicardique de la revendication 1, dans laquelle le microcâble (18) est formé d'une pluralité de brins toronnés ensemble, dont au moins certains sont des brins incorporant une âme en matériau radio-opaque de type platine-iridium ou tantale enveloppée dans une gaine en matériau mécaniquement endurant de type NiTi ou inox, ou inversement.

15. La sonde épicardique de la revendication 1, dans laquelle le microcâble (18) comprend une structure multifilaire revêtue par un matériau isolant, notamment par du parylène ou une gaine de type PET ou PMMA, dans lequel les régions dénudées (20) sont formées en ménageant des ouvertures par ablation le long du microcâble.

16. La sonde épicardique de la revendication 1, dans laquelle l'extrémité du microcâble est dotée d'une aiguille courbe pour le piquage et l'enfouissement du microcâble lors de la pose de la sonde, cette aiguille étant apte à être ultérieurement dissociée du microcâble par découpe.

17. La sonde épicardique de la revendication 16, comprenant un fil résorbable entre l'aiguille et l'extrémité du microcâble.

## Patentansprüche

1. Epikardiale Stimulationssonde (10), die enthält:
- einen Sondenkörper (12) mit einer Hülle aus verformbarem Material, die mindestens einen Verbindungsleiter umschließt;
- auf der proximalen Seite einen Verbinder (14) zum Koppeln mit einem Generator einer aktiven implantierbaren medizinischen Vorrichtung;
- auf der distalen Seite Einrichtungen zur Verankerung an einer Wand des Epikards und einen aktiven Teil, der eine Vielzahl von Stimulationselektroden enthält, die mit der Wand des Epikards in Kontakt kommen oder in sie eindringen kann;
- eine Verteilerbox (16), die am Ende des Sondenkörpers angeordnet ist; und
- ein Netz von elektrisch isolierten, biegsamen Leitern (18), die ausgehend von der Verteilerbox ausstrahlen und sich zwischen einem mit der Verteilerbox verbundenen proximalen Ende und einem von der Verteilerbox entfernten freien distalen Ende erstrecken und im Bereich der Verteilerbox mit einem Verbindungsleiter des Sondenkörpers elektrisch verbunden sind,
wobei die Sonde **dadurch gekennzeichnet ist, dass**:
- die biegsamen Leiter (18) Elemente von der Art Mikrokabel mit einem Durchmesser von höchstens gleich 2 French (0,66 mm) sind;
- jedes Mikrokabel mindestens eine punktuell abisolierte Zone (20) enthält, wobei jede dieser Zonen eine der Stimulationselektroden bildet, wobei die Stimulationselektroden des gleichen Mikrokabels elektrisch miteinander verbunden sind, und
- jedes Mikrokabel mindestens ein längliches Querelement (22; 28) aufweist, das sich unter Bildung eines Winkels mit der Hauptrichtung des Mikrokabels erstreckt und in die Wand des Epikards eindringen kann.

2. Epikardiale Sonde nach Anspruch 1, wobei das mindestens eine längliche Querelement eine von einer Krümmung des Mikrokabels gebildete Verlegeschleife (22) ist.

3. Epikardiale Sonde nach Anspruch 1, wobei das mindestens eine längliche Querelement eine freie Verlängerung (28) ist, die in Abzweigung bezüglich des Mikrokabels geformt wird.

4. Epikardiale Sonde nach Anspruch 1, wobei ein radial distales Ende des mindestens einen länglichen Querelements (22; 28) eine der punktuell abisolierten Zonen trägt, um eine Tiefenstimulation des Epikards zu erlauben.

5. Epikardiale Sonde nach Anspruch 1, wobei jedes Mikrokabel eine Folge von länglichen Teilen aufweist, die sich in der Hauptrichtung des Mikrokabels erstrecken, voneinander durch Compliance-Schleifen (24) getrennt sind, die sich in einer Querrichtung zum Mikrokabel erstrecken und dem Mikrokabel eine Biegsamkeit und eine Dehnbarkeit in Längsrichtung verleihen können.

6. Epikardiale Sonde nach Anspruch 5, wobei die punktuell abisolierten Zonen (20) jedes Mikrokabels an einem radial distalen Ende der Compliance-Schleifen (24) geformt sind, um eine Oberflächenstimulation des Epikards zu erlauben.

7. Epikardiale Sonde nach Anspruch 5, wobei die Compliance-Schleifen (24) und die länglichen Querelemente (22; 28) sich in unterschiedlichen Ebenen (P₁, P₂) erstrecken, die einen Winkel zueinander bilden.

8. Epikardiale Sonde nach Anspruch 1, wobei die Länge jedes Mikrokabels im ausgestreckten Zustand zwischen 5 und 80 mm liegt.

9. Epikardiale Sonde nach Anspruch 1, wobei die Verteilerbox (16) die Einrichtungen zur Verankerung an einer Wand des Epikards trägt.

10. Epikardiale Sonde nach Anspruch 1, wobei die freiliegende Gesamtfläche der punktuell abisolierten Zonen jedes Mikrokabels höchstens 10 mm² beträgt.

11. Epikardiale Sonde nach Anspruch 1, wobei die Länge in Längsrichtung jeder punktuell abisolierten Zone jedes Mikrokabels höchstens 10 mm beträgt.

12. Epikardiale Sonde nach Anspruch 1, wobei der Sondenkörper eine Vielzahl von unterschiedlichen Verbindungsleitern umschließt, und die Sonde eine entsprechende Vielzahl von untereinander elektrisch isolierten und je mit den Verbindungsleitern verbundenen Mikrokabeln aufweist, um eine bipolare oder multipolare Stimulation zwischen punktuell abisolierten Teilen (20, 20') unterschiedlicher Mikrokabel zu erlauben.

13. Epikardiale Sonde nach Anspruch 1, wobei die Sonde eine entsprechende Vielzahl von untereinander elektrisch isolierten Mikrokabeln aufweist, und die Verteilerbox Einrichtungen zur gesteuerten Umschaltung zwischen einerseits einem aus mehreren der Vielzahl von Mikrokabeln ausgewählten Mikrokabel und andererseits einem gemeinsamen Verbindungsleiter des Sondenkörpers aufweist, um eine Auswahl unter den punktförmig abisolierten Teilen (20, 20', 20", 20"') unterschiedlicher Mikrokabel zu erlauben.

14. Epikardiale Sonde nach Anspruch 1, wobei das Mikrokabel (18) von einer Vielzahl von zusammen verdrillten Adern geformt wird, von denen zumindest bestimmte Adern sind, die eine Seele aus röntgendichtem Material von der Art Platin-Iridium oder Tantal enthalten, eingehüllt in eine Hülle aus mechanisch ausdauerndem Material von der Art NiTi oder rostfreier Stahl, oder umgekehrt.

15. Epikardiale Sonde nach Anspruch 1, wobei das Mikrokabel (18) eine mit einem Isoliermaterial, insbesondere aus Parylen oder einer Hülle der Art PET oder PMMA, bedeckte Mehrdraht-Struktur enthält, wobei die abisolierten Bereiche (20) geformt werden, indem Öffnungen durch Abtragung entlang des Mikrokabels ausgespart werden.

16. Epikardiale Sonde nach Anspruch 1, wobei das Ende des Mikrokabels mit einer krummen Nadel zum Einstechen und Verlegen des Mikrokabels beim Einsetzen der Sonde versehen ist, wobei diese Nadel später durch Schneiden vom Mikrokabel getrennt werden kann.

17. Epikardiale Sonde nach Anspruch 16, die einen resorbierbaren Faden zwischen der Nadel und dem Ende des Mikrokabels enthält.

## Claims

1. An epicardial stimulation lead (10), comprising:
- a lead body (12) with a sheath made of a deformable material, enclosing at least one connecting conductor;
- on the proximal side, a connector (14) for the coupling to a generator of an active implantable medical device;
- on the distal side, means for the anchoring to a wall of the epicardium and an active part comprising a plurality of stimulation electrodes, adapted to come into contact with, or penetrate, the epicardium wall;
- a distribution box (16), disposed at the end of the lead body; and
- an array of electrically-insulated flexible conductors (18) radiating from the distribution box and extending between a proximal end connected to the distribution box and a free distal end that is distant from the distribution box and electrically connected to ta connecting conductor of the lead body at the distribution box,
the lead being **characterized in that**:
- said flexible conductors (18) are elements of the micro-cable type of diameter at most equal to 2 French (0.66 mm);
- each micro-cable comprises at least one punctually naked area (20), each of these areas forming one of said stimulation electrodes, the stimulation electrodes of a same micro-cable being electrically connected together, and
- each micro-cable includes at least one transverse elongated element (22; 28) extending so as to form an angle with respect to the main direction of the micro-cable, and adapted to penetrate the epicardium wall.

2. The epicardial lead of claim 1, wherein said at least one transverse elongated element is a burial loop (22) formed by a curvature of the micro-cable.

3. The epicardial lead of claim 1, wherein said at least one transverse elongated element is a free extension (28) formed in parallel with respect to the micro-cable.

4. The epicardial lead of claim 1, wherein a radially distal end of said at least one transverse elongated element (22; 28) carries one of said punctually naked areas, so as to allow a deep stimulation of the epicardium.

5. The epicardial lead of claim 1, wherein each micro-cable includes a succession of elongated portions extending along the main direction of the micro-cable, separated from each other by compliance loops (24) extending in a transverse direction with respect to the micro-cable and adapted to provide the micro-cable with flexibility and extensibility in the longitudinal direction.

6. The epicardial lead of claim 5, wherein the punctually naked areas (20) of each micro-cable are formed at a radially distal end of the compliance loops (24), so as to allow a surface stimulation of the epicardium.

7. The epicardial lead of claim 5, wherein the compliance loops (24) and the transverse elongated elements (22; 28) extend in different respective planes (P₁, P₂) forming an angle between each other.

8. The epicardial lead of claim 1, wherein the length of each micro-cable, at the extended state, is comprised between 5 and 80 mm.

9. The epicardial lead of claim 1, wherein the distribution box (16) carries the means for the anchoring to a wall of the epicardium.

10. The epicardial lead of claim 1, wherein the total exposed surface of the punctually naked areas of each micro-cable is of at most 10 mm².

11. The epicardial lead of claim 1, wherein the length in the longitudinal direction of each punctually naked area of each micro-cable is of at most 10 mm.

12. The epicardial lead of claim 1, wherein the lead body encloses a plurality of distinct connecting conductors, and the lead includes a corresponding plurality of micro-cables electrically insulated from each other and respectively connected to the connecting conductors, so as to allow a bipolar or multipolar stimulation between punctually naked portions (20, 20') of different respective micro-cables.

13. The epicardial lead of claim 1, wherein the lead includes a corresponding plurality of micro-cables electrically insulated from each other, and the distribution box includes controlled switching means between, on the one hand, a micro-cable selected among several of the plurality of micro-cables, and on the other hand, a common connecting conductor of the lead body, so as to allow a selection among the punctually naked portions (20, 20', 20", 20"') of different respective micro-cables.

14. The epicardial probe of claim 1, wherein the micro-cable (18) is formed of a plurality of cords stranded together, at least some of which are cords incorporating a core made of a radio-opaque material of the platinum-iridium or tantalum type enveloped with a sheath made of a mechanically tough material of the NiTi or stainless steel type, or vice versa.

15. The epicardial lead of claim 1, wherein the micro-cable (18) comprises a multi-wire structure coated with an insulating material, in particular with parylene or a sheath of the PET or PMMA type, wherein the naked areas (20) are formed by arranging openings by ablation along the micro-cable.

16. The epicardial lead of claim 1, wherein the end of the micro-cable is provided with a curved needle for the picking and the burial of the micro-cable during the installation of the lead, this needle being adapted to be lately dissociated from the micro-cable by cutting.

17. The epicardial lead of claim 16, comprising a resorptive wire between the needle and the end of the micro-cable.
